(19) Europäisches Patentamt / European Patent Office / Office européen des brevets

(11) **EP 3 714 031 B1**

(12) **FASCICULE DE BREVET EUROPÉEN**

(45) Date de publication et mention de la délivrance du brevet:
**05.01.2022 Bulletin 2022/01**

(21) Numéro de dépôt: **17817785.3**

(22) Date de dépôt: **23.11.2017**

(51) Int Cl.:
*C12M 1/107* *(2006.01)*    *C12M 1/00* *(2006.01)*

(86) Numéro de dépôt international:
**PCT/FR2017/053239**

(87) Numéro de publication internationale:
**WO 2018/096287 (31.05.2018 Gazette 2018/22)**

(54) **DISPOSITIF ET PROCÉDÉ DE MÉTHANISATION**

METHANISIERUNGSVORRICHTUNG UND -VERFAHREN

METHANATION DEVICE AND METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**30.09.2020 Bulletin 2020/40**

(73) Titulaire: **Arkolia Energies**
**34130 Mudaison (FR)**

(72) Inventeurs:
• **BORNET, Nicolas**
**34130 Mudaison (FR)**
• **BLANC, Frédéric**
**34130 Mudaison (FR)**

• **GIBOULET, Florie**
**34130 Mudaison (FR)**
• **GUENDOUZ, Amel**
**34130 Mudaison (FR)**
• **GUILLAUME, Sophie**
**34130 Mudaison (FR)**
• **HATTOU, Stéphane**
**34130 Mudaison (FR)**

(74) Mandataire: **Cornuejols, Georges**
**Cassiopi**
**230 Avenue de l'Aube Rouge**
**34170 Castelnau-le-Lez (FR)**

(56) Documents cités:
**EP-A1- 1 811 015        WO-A1-2014/027165**
**DE-A1-102008 058 114**

**Description**

DOMAINE TECHNIQUE DE L'INVENTION

La présente invention vise un dispositif et un procédé de méthanisation. Elle s'applique, notamment, au domaine de la méthanisation. Plus particulièrement, la présente invention s'applique aux réacteurs et aux réactions de méthanisation dans lesquels la concentration d'une atmosphère en ammoniac est contrôlée. Elle s'applique à la méthanisation continue en voie sèche, ainsi qu'à la méthanisation continue en voie liquide.

ETAT DE LA TECHNIQUE

Parmi les gisements convenant à la méthanisation, il existe plusieurs sources d'azote, à savoir :

- l'azote minéral préférentiellement issu des urines (présents dans les fumiers et lisiers)
- l'azote organique issu des protéines contenues dans la matière organique.

Une partie de l'ammoniac est produit au cours des étapes de réduction de la matière organique. La composition biochimique d'une protéine peut s'écrire $C_{53}H_6O_{28}N_{12}S_3$.

Selon l'équation de Buswell, il est alors possible d'estimer les taux d'ammoniac dans le milieu (Buswell, A. M. and H. F. Mueller (1952) Mechanism of methane fermentation. Industrial and Engineering Chemistry 44(3): 550-552).

$$CcH_hO_oN_nS_s + 1/4(4c\text{-}h\text{-}2o+3n+2s)\,H_2O = 1/8(4c+h\text{-}2o\text{-}3n\text{-}2s)CH_4 + 1/8(4c\text{-}h+2o+3n+2s)CO_2 + nNH_3 + sH_2S \qquad (A)$$

En fonction des conditions physico-chimiques du milieu, telles que le potentiel hydrogène (d'acronyme « pH ») et la température en degrés Celsius, l'ammoniac se retrouve sous forme libre ($NH_3$) ou ionisée ($NH_4+$). Chacune de ces formes semble avoir une action inhibitrice sur le procédé de méthanisation. Cette inhibition intervient à différents stades de la réaction, et peut impacter une ou plusieurs voies métaboliques. Le mécanisme d'inhibition de la chaine de réaction de méthanisation reste mal connu. EP1811015 divulgue (fig. 1) un dispositif pour la production de biogaz comprenant un moyen pour extraire le biogaz produit dans un bioréacteur et le réintroduire à l'aide d'une cheminée et des embouts en bas du bioréacteur.

De nombreuses études, telles que celles de Kayhanian en 1999 et Sprott et Patel en 1986, mettent en cause le $NH_3$ sous sa forme libre, qui par diffusion passive au travers des membranes, entraînerait un changement de pH intracellulaire, induisant un besoin énergétique important pour la maintenance cellulaire, accompagné d'un épuisement intracellulaire du potassium ionisé. Cette inhibition serait donc non exclusive.

L'étude réalisée par Shumei Gao en 2015, a montré que l'étape d'hydrolyse de la réaction de méthanisation était peu impactée par une concentration en $NH_3$ libre de concentration inférieure à 0,5 gramme par litre (g/L). En revanche, l'étape de méthanogenèse semble prendre préférentiellement la voie hydrogénotrophe. Cette versatilité a été mise en évidence par l'augmentation d'activité du cofacteur 420, transporteur d'électron spécifique aux populations hydrogénotrophe.

Si la concentration en ammoniac ionisée augmente au-delà de 3 g/L, l'ammoniac ionisée peut devenir inhibiteur de la réaction de méthanogenèse. On parle alors d'intoxication à l'ammoniac ($NH_3$) ou alcalose.

Le $NH_3$ inhiberait les bactéries acidogènes et acétogènes ; les produits d'hydrolyse, tels les acides aminés et les acides gras volatils, s'accumulent ce qui peut conduire à une chute du pH dans le digesteur, ou acidose.

L'inhibition peut également intervenir au niveau de la voie de méthanogenèse, ce qui se traduit par une accumulation en acétate et une diminution du pH.

En règle générale, l'inhibition se traduit par une diminution plus ou moins importante de la quantité de biogaz produit.

D'un autre côté, l'ammoniac ionisée est à l'origine de la formation des hydrogénocarbonates, donc du pouvoir tampon du milieu de fermentation anaérobie. A pH basique, les acides gras volatils sont préférentiellement sous leur forme ionisée, atténuant ainsi leur effet inhibiteur. La diffusion des acides gras volatils sous la forme non ionisée à travers les membranes cellulaires, induit une concentration importante dans le milieu intracellulaire, créant ainsi une inhibition des microorganismes. L'ammoniac, en deçà d'une concentration de 3 g/L, est donc à ce titre bénéfique.

Il est donc fondamental de réguler la quantité d'ammoniac présente dans le digesteur. Il est classiquement admis que les facteurs sur lesquels il est possible d'agir, pour réguler la concentration en ammoniac, sont :

- La nature des substrats introduits :

- une matière présentant un rapport carbone sur azote, dit « C/N » faible, et/ou un taux d'azote organique, dit « $N_{org}$ », élevé, conduit à un taux d'ammoniac important dans le réacteur de méthanisation (ou méthaniseur), en raison d'une dégradation normale de ce composé azoté qui est un processus classique de minéralisation de la matière dégradée ;
- une matière déjà riche en ammoniac conduit aussi par action additive à un taux élevé d'ammoniac dans le méthaniseur ;

- la dilution du méthaniseur, par de l'eau fraîche, qui permet de diminuer les taux d'ammoniac dans le méthaniseur,
- la recirculation des liquides issus de la méthanisation, qui solubilisent une part importante de l'ammoniac, en effet, sans traitement spécifique, cela conduit à une augmentation de la concentration en ammoniac par action additive.
- le temps de séjour moyen hydraulique : plus il est important, et plus on observe une minéralisation complète de l'azote organique $N_{org}$, induisant ainsi une augmentation de la concentration en ammoniac dans le méthaniseur.
- le couple température et pH : une température de 55°C et un pH de 8 favorisent la forme $NH_3$, nocive pour les microorganismes, alors qu'une température de 37°C et un pH inférieur à 7,4 conduisent à la forme $NH_4$, moins inhibitrice.

La stabilité d'un digesteur anaérobie dépend donc du contrôle de la concentration en ammoniac.

OBJET DE L'INVENTION

La présente invention vise à remédier à tout ou partie de ces inconvénients. Notamment, la présente invention vise à contrôler la concentration en ammoniac dans une atmosphère d'un compartiment de méthanisation pour améliorer la rapidité et la stabilité dans le temps d'une réaction chimique. La présente invention vise également à orienter la réaction de méthanisation pour obtenir la forme désirée d'ammoniac.

A cet effet, selon un premier aspect, la présente invention vise un dispositif de méthanisation, qui comporte :

- un réacteur de méthanisation comportant au moins un compartiment dans lequel est inséré un substrat en fermentation, produisant une atmosphère comportant au moins du méthane et de l'ammoniac,
- une unité d'extraction de l'atmosphère d'au moins un compartiment, l'unité comportant :

  - un premier conduit d'extraction de l'atmosphère d'au moins un compartiment,
  - une colonne de lavage dans laquelle débouche le premier conduit et qui comporte au moins un injecteur d'un liquide d'épuration,
  - au moins un deuxième conduit d'extraction du gaz lavé dont la concentration en ammoniac a diminué et d'injection dudit gaz dans au moins un compartiment.

Grâce à ces dispositions, l'équilibre de l'ammoniac dissous est régulé par actions simultanées sur :

- la température du réacteur,
- le pH du réacteur et
- la pression partielle en ammoniac gaz dans l'atmosphère du réacteur.

Dans des modes de réalisation, le conduit d'injection présente au moins un orifice d'injection du gaz proche du fond d'un compartiment.
Ces modes de réalisation permettent d'injecter le gaz dont la concentration en ammoniac est diminuée au fond du compartiment, pour remuer le substrat en fermentation et traverser ce substrat selon un procédé dénommé « bullage ».
Dans des modes de réalisation, le liquide d'épuration comporte de l'acide sulfurique, nitrique ou phosphorique.
L'avantage de l'acide est de transformer l'ammoniac de l'atmosphère extraite en sels d'ammonium de type sulfate d'ammonium, qui est un engrais, par réaction avec l'acide.
Dans des modes de réalisation, le réacteur de méthanisation comporte au moins deux compartiments, chaque compartiment comportant un conduit d'injection de gaz pour lequel la quantité de gaz injecté dans chaque compartiment est régi par une vanne, chaque vanne étant pilotée indépendamment.
Grâce à ces dispositions, les réacteurs comprenant plusieurs compartiments, chaque compartiment réalisant une étape différente de la réaction de méthanisation. Les températures et les pH peuvent être adaptés dans les différents compartiments en fonction de ces étapes, la concentration en ammoniac de l'atmosphère d'au moins un compartiment étant régulée de manière indépendante.
Dans des modes de réalisation, le dispositif comporte un capteur de taux d'ammoniac dans la matière en fermentation et une unité de contrôle configurée pour déclencher le fonctionnement de l'unité d'extraction pour réduire la concentration en ion ammonium lorsque la concentration captée est supérieure 4 g/L de matière en fermentation.

Dans des modes de réalisation, l'unité de contrôle est configurée pour déclencher le fonctionnement de l'unité d'extraction pour réduire la concentration en ion ammonium lorsque la concentration captée est supérieure 3 g/L de matière en fermentation.

Dans des modes de réalisation, le dispositif comporte une pluralité de compartiments de fermentation, dont les atmosphères sont rendues indépendantes en étant séparées par au moins une cloison.

Dans des modes de réalisation, le dispositif comporte un piston pour déplacer la matière en fermentation d'un compartiment à un autre.

Dans des modes de réalisation, le dispositif comporte une matière à fermenter dont le taux de matière sèche est supérieur à 15 %.

Dans des modes de réalisation, le dispositif comporte une matière à fermenter dont le taux de matière sèche est supérieur à 20 %.

Dans des modes de réalisation, le dispositif comporte au moins une cheminée descendant dans la matière en fermentation pour injecter le gaz lavé dans un compartiment d'où le gaz à laver a été extrait par l'unité d'extraction.

Dans des modes de réalisation, le dispositif comporte un moyen de mise sous pression du gaz à injecter par une cheminée à une pression comprise entre 5 et 8 bars.

Dans des modes de réalisation, l'unité d'extraction est configurée pour extraire une partie de l'atmosphère dans un compartiment où se trouvent la majorité des bactéries acidogènes.

Dans des modes de réalisation, l'unité d'extraction est configurée pour extraire une partie de l'atmosphère dans un compartiment où se trouvent la majorité des bactéries acétogènes.

Dans des modes de réalisation, l'unité d'extraction est configurée pour extraire une partie de l'atmosphère dans un compartiment où est effectuée la méthanogenèse.

Dans des modes de réalisation, au moins un compartiment dans lequel l'unité d'extraction extrait une partie de l'atmosphère présente une température comprise entre 45°C et 60 °C.

Dans des modes de réalisation, au moins un compartiment dans lequel l'unité d'extraction extrait une partie de l'atmosphère présente un potentiel hydrogène compris entre 7,5 et 8,7.

Dans des modes de réalisation, au moins un compartiment dans lequel l'unité d'extraction extrait une partie de l'atmosphère présente un potentiel hydrogène compris entre 7,9 et 8,7.

Dans des modes de réalisation, au moins un compartiment dans lequel l'unité d'extraction extrait une partie de l'atmosphère présente un titre en méthane compris entre 52% et 65%.

Ces compartiments bénéficient particulièrement de la régulation du taux d'ammoniac offerte par la mise en oeuvre de la présente invention.

Selon un deuxième aspect, la présente invention vise un procédé de méthanisation, qui comporte les étapes suivantes :

- réaction de méthanisation par fermentation d'un substrat dans au moins un compartiment, la réaction produisant une atmosphère comportant au moins du méthane et de l'ammoniac,
- extraction de l'atmosphère d'au moins un compartiment,
- lavage de l'atmosphère extraite par injection d'un liquide d'épuration,
- extraction du gaz lavé dont la concentration en ammoniac a diminué et
- injection dudit gaz dans au moins un compartiment.

Les buts, avantages et caractéristiques particulières du procédé objet de la présente invention étant similaires à ceux du dispositif objet de la présente invention, ils ne sont pas rappelés ici.

BREVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :

- la figure 1 représente, schématiquement, un premier mode de réalisation particulier du dispositif objet de la présente invention,
- la figure 2 représente, schématiquement et sous forme d'un logigramme, une succession d'étapes particulière du procédé objet de la présente invention,
- la figure 3 représente, schématiquement, une unité de méthanisation en voie sèche,
- la figure 4 représente, schématiquement, un laveur de gaz et
- la figure 5 représente un dispositif multi compartiments doté de capteurs et de moyens de contrôle.

## DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse. Par ailleurs, chaque paramètre d'un exemple de réalisation peut être mis en oeuvre indépendamment d'autres paramètres dudit exemple de réalisation.

On note dès à présent que les figures ne sont pas à l'échelle.

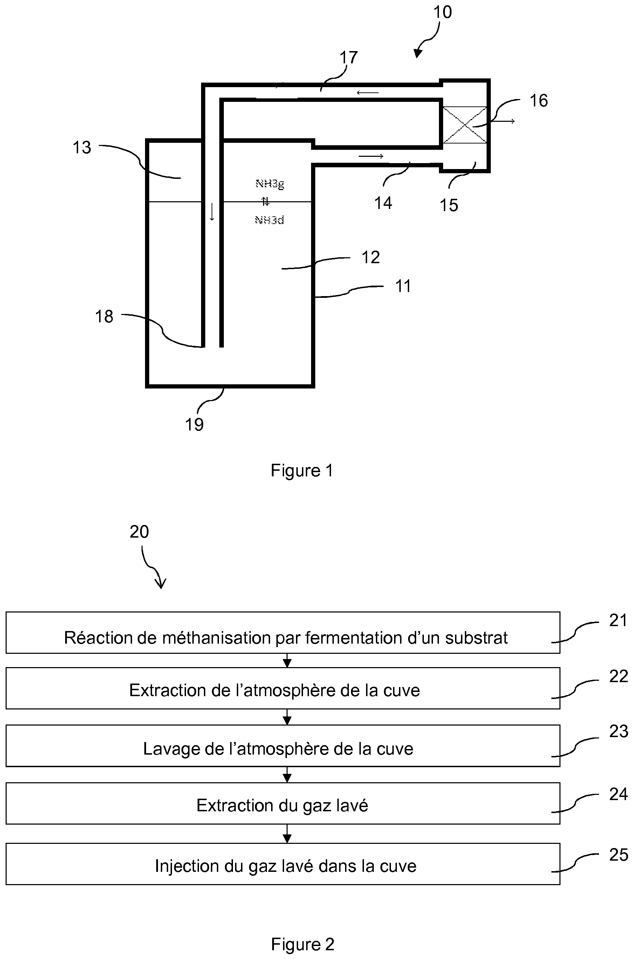

On observe, sur la figure 1, qui n'est pas à l'échelle, une vue schématique d'un mode de réalisation du dispositif 10 objet de la présente invention.

Le dispositif de méthanisation 10 comporte :

- un réacteur de méthanisation comportant un compartiment 11 dans lequel se trouve un substrat 12 en fermentation, produisant une atmosphère 13 comportant au moins du méthane et de l'ammoniac,
- une unité d'extraction de l'atmosphère du compartiment 11, l'unité comportant :

    - un premier conduit d'extraction 14 de l'atmosphère du compartiment 11,
    - une colonne de lavage 15 dans laquelle débouche le premier conduit 14 et qui comporte au moins un injecteur 16 d'un liquide d'épuration,
    - au moins un deuxième conduit d'extraction 17 du gaz lavé dont la concentration en ammoniac a diminué et d'injection dudit gaz dans le compartiment 11.

Le substrat 12 comporte des bactéries pour le procédé de fermentation. La température, le potentiel hydrogène et le temps de séjour du substrat dans le compartiment sont contrôlés et sont maintenus dans un intervalle de tolérance défini par des valeurs limites prédéterminées. Un tel contrôle oriente la réaction de méthanisation pour former de l'ammoniac sous forme $NH_3$. Le contrôle est effectué dans chaque compartiment du réacteur, notamment dans le compartiment d'hydrolyse.

L'unité d'extraction de l'atmosphère du compartiment 11 est configurée pour réguler la concentration en ammoniac de l'atmosphère de chaque compartiment. La régulation peut être effectuée par un asservissement d'une vanne sur le conduit d'extraction 14 et 17 en fonction de la concentration en ammoniac. Cette concentration peut être soit estimée par calcul réalisé à partir des conditions de température et de pH (selon les équilibres acido-basiques décrits ci-dessous), soit mesurée directement dans le liquide ou le gaz. L'asservissement peut être réalisé par une unité de commande. L'asservissement peut également dépendre du rapport de concentration en méthane ($CH_4$) sur la concentration en ammoniac ($NH_3$) devant être maintenu proche d'une valeur limite prédéterminée. Le rapport de concentration en méthane ($CH_4$) sur la concentration en ammoniac ($NH_3$) peut être remplacé par le rapport de concentration en carbone (C) sur la concentration en azote (N).

Préférentiellement, lorsque le réacteur de méthanisation comporte au moins deux compartiments 11, chaque compartiment 11 comporte un conduit d'injection 17 de gaz pour lequel la quantité de gaz injecté dans chaque compartiment 11 est régi par une vanne, chaque vanne étant pilotée indépendamment. Le pilotage de chaque vanne peut correspondre au mode de réalisation décrit ci-dessus.

Dans des modes de réalisation préférentiels, la pression dans les conduits d'extraction 14 et 17 est égale à la pression de l'atmosphère 13 du compartiment 11. Alternativement, la pression dans au moins un conduit d'extraction, 14 et/ou 17 est inférieure à la pression de l'atmosphère 13 du compartiment 11. Ou, la pression dans au moins un conduit d'extraction 14 et/ou 17 est supérieure à la pression de l'atmosphère 13 du compartiment 11.

La pression dans la colonne de lavage peut également être modifiée tels que les gaz dans la colonne de lavage sont comprimés par rapport à l'atmosphère 13 du compartiment 11. Ces modes de réalisation permettent d'éliminer au moins partiellement le dioxyde de carbone ($CO_2$) dans l'atmosphère du compartiment 11. Notamment, l'élimination du dioxyde de carbone est améliorée lorsque le liquide d'épuration est de l'eau. Une telle élimination du dioxyde de carbone est une méthode de régulation du potentiel hydrogène dans le compartiment 11. Donc dans ces modes de réalisation, la concentration en ammoniac et la concentration en dioxyde de carbone sont régulées simultanément.

Dans des modes de réalisation, la température du liquide d'épuration est optimisée pour laver le gaz plus efficacement.

Dans des modes de réalisation, le conduit d'injection 17 présente un orifice d'injection 18 du gaz proche du fond 19 du compartiment 11 et donc dans le substrat en fermentation 12. L'injection du gaz proche du fond 19 du compartiment 11 permet de remuer le substrat avec un gaz dons la concentration en ammoniac est diminuée.

Dans des modes de réalisation, le conduit d'injection 17 présente un orifice 18 dans l'atmosphère 13 du compartiment 11. Les modes de réalisation de l'injection du gaz peuvent être combinées et l'unité de lavage peut comprendre au moins deux conduits d'injection 17, l'un présentant un orifice d'injection dans l'atmosphère du compartiment 11 et l'autre présentant un orifice d'injection proche du fond 19 du compartiment 11.

Dans des modes de réalisation, le liquide d'épuration comporte de l'acide sulfurique, nitrique ou phosphorique pour transformer l'ammoniac lavé en engrais. Dans d'autres modes de réalisation, on utilise de l'acide phosphorique, de l'acide nitrique ou de l'eau.

Concernant l'ammoniac, l'équilibre ionique en solution dans le réacteur est régi par l'équation :

$$NH_{3d} + H_2O \leftrightarrows NH_4OH \leftrightarrows NH_4^+ + OH^- \qquad (B)$$

Par exemple, en se plaçant à 55°C et à un pH supérieur à 7,5, on oriente l'équilibre vers la forme $NH_3$ dissout ($NH_{3d}$) dans le réacteur. La forme dissoute est inhibitrice au-delà d'un certain seuil de concentration dans le liquide.

Il convient donc de faire passer l'ammoniac du liquide vers le gaz, selon l'équilibre :

$$NH_{3d} \leftrightarrows NH_{3g} \qquad (C)$$

Selon la loi de Henry, la concentration maximale d'un gaz en solution, en équilibre avec une atmosphère contenant ce gaz, est proportionnelle à la pression partielle de ce gaz en ce point :

$$C_{NH3d}^s = p_{NH3g} \times H_{NH3} \qquad (D)$$

avec

$C_{NH3d}^s$ : la concentration maximale (à saturation) de l'ammoniac dans le liquide
$p_{NH3g}$ : la pression partielle de l'ammoniac dans le gaz
$H_{NH3}$ : la constante de Henry pour l'ammoniac.

Selon l'équation D, il est donc nécessaire de diminuer la pression partielle de l'ammoniac dans l'atmosphère 13 du compartiment 11 du réacteur de méthanisation. Cette opération est effectuée par recirculation du biogaz produit dans le compartiment 11 du réacteur de méthanisation, avec élimination de l'ammoniac $NH_{3g}$ dans la boucle de recirculation.

Une partie du biogaz produit et composant l'atmosphère 13 du compartiment 11 est traitée dans l'unité de lavage qui est configurée pour éliminer du $NH_{3g}$. Puis le biogaz est réinjecté avec une concentration et $NH_3$ diminuée dans le compartiment 11. Cette opération a pour effet de réduire la quantité de $NH_3$ sous forme dissoute par abaissement de la pression partielle dans l'atmosphère 13 du compartiment 11, le $NH_3$ prend une forme gazeuse. L'atmosphère 13 ensuite enrichi en $NH_{3g}$ est de nouveau traité dans l'unité de lavage, et le cycle est reproduit jusqu'à obtenir les concentrations désirées en ammoniac dans le substrat 12 et dans l'atmosphère 13.

La figure 2 représente, schématiquement, un procédé 20 de méthanisation, comportant les étapes suivantes :

- réaction 21 de méthanisation par fermentation d'un substrat 12 dans un compartiment 11, la réaction produisant une atmosphère 13 comportant au moins du méthane et de l'ammoniac,
- extraction 22 de l'atmosphère 13 du compartiment 11,
- lavage 23 de l'atmosphère extraite par injection d'un liquide d'épuration,
- extraction 24 du gaz lavé dont la concentration en ammoniac a diminué et
- injection 25 dudit gaz dans le compartiment 11.

Le procédé d'élimination de l'ammoniac $NH_{3g}$ de l'atmosphère 13 du compartiment 11 est détaillé ci-après.

La colonne de lavage 15 réalise l'étape de lavage 23, par exemple avec un liquide d'épuration comportant de l'acide sulfurique. Le $NH_{3g}$ est transformé en sulfate d'ammonium, qui est un engrais, puis le sulfate d'ammonium est récupéré en fond de colonne 15. Le gaz épuré de l'ammoniac est récupéré en tête de colonne de lavage avant d'être réinjecté dans le compartiment 11.

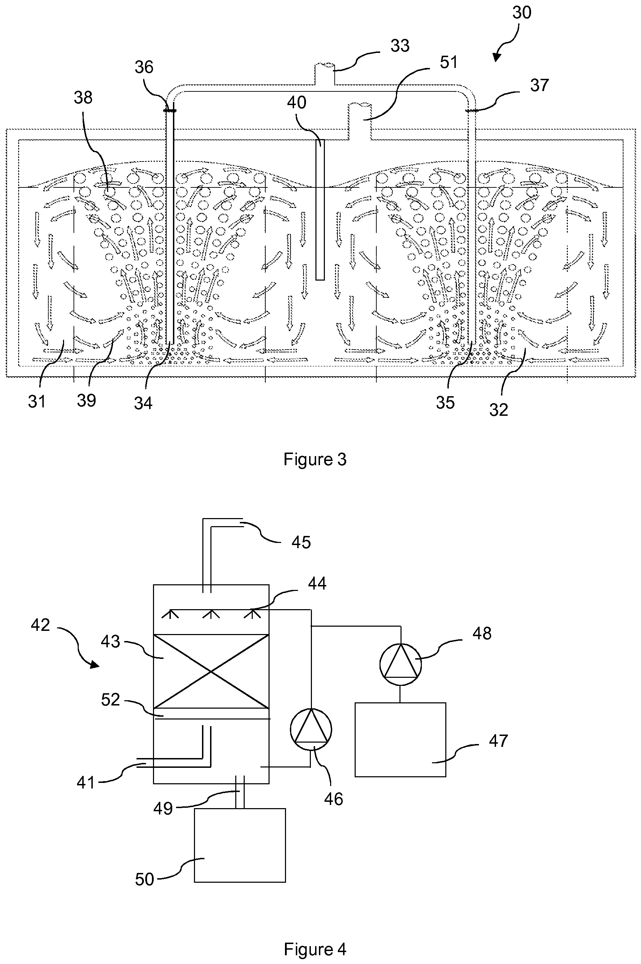

On observe, en figure 4, un laveur comportant, de l'amont vers l'aval du déplacement du gaz à laver :

- une entrée 41 ; et
- un fut de colonne 42 de lavage comportant

  - un garnissage 43,
  - des asperseurs 44 et
  - une sortie 45.

A contrecourant du gaz, circule un liquide pompé par une pompe principale 46 en base de la colonne de lavage 42 et injecté dans les asperseurs 44. Une pompe secondaire 48 pompe de l'acide dans un réservoir 47 et l'additionne au liquide en circulation.

Une sortie 49 en bas de la colonne de lavage 42 permet de récupérer le liquide dans un compartiment 50. Ce liquide dans le compartiment 50 peut notamment servir d'engrais.

La colonne de lavage 42 prend ici la forme d'une colonne à garnissage, qui comporte généralement les éléments suivants :

- une grille 52 support de garnissage permettant de supporter le poids du garnissage et du liquide retenu sur ce dernier et assurant une distribution des liquides en bas de colonne.
- un dévésiculeur (non représenté) pour l'élimination des gouttes de liquides qui pourraient être entraînées par le gaz.

Le fonctionnement est ici à contre-courant : le gaz est ascendant et le liquide de lavage s'écoule par gravité sur le garnissage. Le choix du garnissage, élément essentiel de ce type de laveur, est dicté par la surface de contact offerte entre le gaz et le liquide utilisé, le calcul des pertes de charge et son prix. Les garnissages peuvent être de formes variées (anneaux, selles...), de matériaux différents (céramique, verre, métal...) et être rangés ou disposés en vrac. Dans la colonne illustrée en figure 4, de l'atmosphère prélevée dans l'unité de méthanisation entre par une entrée inférieure et remonte le fut de colonne de lavage 42 jusqu'à la sortie de gaz lavée 45, après avoir traversé, successivement, la grille support et le garnissage 43. En sens inverse, la solution de lavage pénètre dans le fut de colonne de lavage 42 par une entrée supérieure, traverse le garnissage 43 et la grille support avec d'être évacué par l'intermédiaire d'une vanne (non représentée), vers une sortie de recirculation ou de compartiment.

Selon les conditions d'utilisation de la colonne de lavage 15, tout ou partie du $CO_2$ présent dans l'atmosphère 13 extraite peut être éliminé. Un fonctionnement avec uniquement de l'eau, ou sous pression, entraîne un appauvrissement de l'atmosphère en $CO_2$.

Préférentiellement, selon les équations d'équilibres chimiques et thermodynamiques décrites plus haut, le procédé d'élimination de l'ammoniac est effectué dans les conditions suivantes :

- la température dans le réacteur 12 est comprise entre 45°C et 60°C et préférentiellement entre 50°C et 60 °C.
- le potentiel hydrogène dans le réacteur 12 est compris entre 7,5 et 8,5.

Habituellement, la combinaison de ces conditions de fonctionnement est évitée dans les procédés de méthanisation, notamment les points de fonctionnement avec une température supérieure à 55°C et potentiel hydrogène supérieur à 8, puisque ces points de fonctionnement sont susceptibles d'être fortement inhibiteurs du procédé de méthanisation.

Le procédé 20 objet de la présente invention, d'élimination in-situ de l'ammoniac permet de travailler avec de telles températures et des tels potentiels hydrogènes, permettant de maximiser la cinétique du procédé de méthanisation.

Les temps de séjour du substrat dans un compartiment avec le procédé 20 objet de la présente invention sont :

- pour un réacteur fonctionnant en mode piston ou semi-piston, c'est-à-dire pour un procédé de méthanisation en continu, le temps de séjour du substrat dans le compartiment d'hydrolyse est compris entre 0,5 et 5 jours et le temps de séjour du substrat dans le compartiment de digestion est compris entre 5 et 25 jours ; et
- pour un réacteur fonctionnant en infiniment mélangé, le temps de séjour du substrat dans le compartiment de digestion est compris entre 30 et 60 jours.

Dans un réacteur fonctionnant en mode piston ou semi-piston, l'équilibre acido-basique du procédé de méthanisation 20 est contrôlé en temps réel par un asservissement (tel que décrit ci-dessus), en agissant sur une zone spécifique au sein de l'écoulement. Un cloisonnement du substrat 12 et/ou de l'atmosphère 13, le long du flux du piston, permet d'agir notamment de manière indépendante soit sur le compartiment d'hydrolyse, soit sur un autre compartiment en aval de l'hydrolyse. Le pilotage de l'équilibre acido-basique peut aussi être optimisé par l'élimination dans l'atmosphère d'une partie du dioxyde de carbone.

La présente invention peut être mise en oeuvre dans une unité de méthanisation en voie épaisse, par exemple telle que décrite dans la demande internationale PCT/FR2013/051938 publiée sous la référence WO 2014/027165, incorporé ici par référence, ou dans une unité de méthanisation plus classique biphasés en voie liquide dite « en infiniment mélangé ».

On observe, en figure 3, une unité de méthanisation en voie épaisse 30, dont seules deux compartiments 31 et 32, séparés par une cloison 40, sont représentés et seules une cheminée, respectivement 34 et 35, est implantée dans chaque compartiment.

Bien que ce mode de réalisation présente des cheminées descendant du plafond du compartiment, la présente invention est aussi bien mise en oeuvre avec des buses ou des tuyauteries d'injection de gaz partant d'une paroi latérale ou du plancher d'un compartiment.

Une entrée de gaz 33 distribue du gaz, par l'intermédiaire de vannes 36 et 37 commandées indépendamment, dans les cheminées 34 et 35. L'injection de gaz sous pression provoque la remontée de bulles de gaz 38, qui entraîne la matière dans un mouvement de convection, par exemple en suivant le chemin indiqué par les flèches 39. Une sortie de gaz 51 permet le prélèvement de parties des atmosphères des différents compartiments.

Dans l'unité de méthanisation en voie épaisse, la taille des installations requises nécessite la plupart du temps un volume de méthaniseur le plus faible possible, impliquant d'une part une siccité élevée (supérieure à 15 % et préférentiellement à 17 %) et, d'autre part, une température de fonctionnement thermophile (environ 55°C). Ces conditions de fonctionnement (concentrations et température élevées) rendent plus fragile la flore bactérienne vis-à-vis de l'ammoniac, composé inhérent au process de méthanisation. Cela limite fortement l'utilisation de substrats/déchets azotés, et par voie de conséquence le développement de la filière.

Les procédés biphasé voie liquide en infiniment mélangé sont des procédés à faible siccité (inférieure à 10 %). Ces procédés nécessitent naturellement une recirculation importante des jus en sortie de méthaniseur, pour minimiser les quantités d'eau de dilution et les rejets aqueux en sortie du procédé de méthanisation. Ces procédés sont les plus utilisés en milieu agricole. Néanmoins, afin de pouvoir utiliser des substrats fortement azotés (comme les fumiers d'engraissement par exemple), il est nécessaire d'introduire des co-substrats carbonés, et de minimiser la recirculation des jus issus du procédé, augmentant ainsi les quantités d'eau de dilution et de rejets aqueux.

La présente invention présente une solution industrielle de gestion de l'ammoniac sur ces deux types de procédé.

Parmi les gisements méthanisables, il existe plusieurs sources d'azote :

- l'azote minéral préférentiellement issu des urines (principalement présent dans les fumiers et lisiers) et
- l'azote organique issu des protéines contenues dans la matière organique.

Une importante partie de l'ammoniac est produit au cours des étapes de réduction de la matière organique. En fonction des conditions physico-chimiques du milieu (pH et Température) l'ammoniac se retrouve sous forme libre ($NH_3$) ou ionisée, ion ammonium ($NH_4^+$). Chacune de ces formes possède une action inhibitrice sur le procédé de méthanisation. Néanmoins, la forme libre $NH_3$ est reconnue comme étant la plus nocive. Cette inhibition intervient à différents stades de la réaction, et peut impacter une ou plusieurs voies métaboliques. Si la concentration en ion ammonium augmente au-delà de 3 g/L, il peut devenir inhibiteur de la réaction de méthanogenèse. On parle alors d'intoxication à l'ammoniac ($NH_3$) ou alcalose.

La concentration est mesurée dans la partie liquide de la matière en fermentation : le taux de 3 g/L d'ammoniac est une valeur en deçà de laquelle il y a peu de problèmes d'inhibition. Au-delà, les risques sont augmentés mais il reste néanmoins possible de fonctionner à des valeurs plus élevées. Préférentiellement, l'asservissement du taux d'ammoniac vise à ce qu'il reste rester inférieur à 4 g/L et, encore plus préférentiellement, inférieur à 3 g/L.

La présente invention permet de réguler la quantité d'ammoniac (forme $NH_3$ ou $NH_4^+$) présente dans le digesteur. En effet, la stabilité d'un digesteur anaérobie dépend du contrôle de la concentration de l'ammoniac.

La présente invention permet donc d'utiliser des substrats présentant un déséquilibre sur l'azote total, dans les conditions d'un procédé voie épaisse (forte siccité et température élevée). Cela permet, d'une part, le développement de ce type de procédé et, d'autre part, d'ouvrir le marché actuel à de nombreux substrats/déchets pour l'instant peu utilisés dans ces procédés (car entraînant une inhibition à l'ammoniac). Cela réduit (voire élimine) aussi l'utilisation d'eau de dilution mise en oeuvre sur les procédés actuels, minimisant fortement les rejets « aqueux » en sortie de méthaniseur. Les deux avantages précités sont de nature à favoriser le développement des procédés de méthanisation en voie épaisse.

La présente invention permet aussi de maîtriser le taux de fertilisants (notamment l'azote) lorsque le produit (digestat ou jus de presse) est utilisé en épandage agricole.

La présente invention permet d'améliorer la qualité du produit issus du méthaniseur afin d'élargir les destinations des produits sortants comme engrais commerciaux.

Le procédé de méthanisation en voie épaisse, par exemple telle que décrite dans la demande internationale PCT/FR2013/051938 se caractérise par :

- Un fermenteur compartimenté permettant une séparation des différentes phases de la méthanisation (dans l'ordre, hydrolyse et acidogenèse, acétogenèse et méthanogenèse). Le fermenteur, subdivisé en secteurs d'agitation, correspond ainsi à autant de « réacteurs indépendants » que de secteurs d'agitations, chacun recevant un substrat fermenté venant du secteur précédent dans un inoculum approprié. Cette cascade de réacteurs permet, à la fois, d'optimiser la vitesse de réaction des bactéries et de piloter précisément l'ensemble du fermenteur.
- Un système d'agitation fait de cheminées partant du toit du fermenteur jusqu'au plancher de celui-ci et laissant passer du biogaz sous pression, à un débit élevé et variable, dépendant de la viscosité du milieu. Cette introduction de gaz est réalisée de manière séquentielle durant quelques secondes en générant un balayage du fond suivi d'un mouvement convectif nécessaire à l'homogénéisation du substrat en fermentation. Les cheminées sont réparties sur l'ensemble du fermenteur et déterminent ainsi des secteurs d'agitation. Le brassage de la matière par l'injection du biogaz sous pression par l'intermédiaire des cheminées permet un fonctionnement avec un taux élevé de matière sèche (entre 20% et 30%) selon le substrat et ne laisse pas de volumes morts. Le taux de matière sèche est un indicateur peu fiable de la viscosité. Plus le substrat contient des éléments inertes (bois, verres, cailloux, plastiques) et plus le taux de matière sèche admissible est élevé. Chaque secteur d'agitation est équipé de deux à trois cheminées qui reçoivent du biogaz détendu à une pression entre 5 et 8 bars générant un débit important sur quelques secondes de l'ordre de 8000 $m^3.h^{-1}$.

- Par une cinétique de dégradation rapide améliorée par l'hydrolyse thermo-enzymatique et le cheminement piston du substrat en fermentation permettant de réduire le temps de séjour hydraulique et donc le volume des fermenteurs.
- Par le pilotage de l'équilibre acido-basique rendu possible à la fois par les conditions thermodynamiques des compartiments et l'évolution sectorielle du substrat.

La gestion de la concentration de l'ammoniac dans le fermenteur peut être envisagée par action sur des secteurs identifiés (derniers secteurs du réacteur piston, notamment). L'équilibre acido-basique peut alors être mieux contrôlé le long du flux piston, optimisant ainsi dans l'espace la production de biogaz.

L'équilibre ionique en solution dans le réacteur est régi par l'équation :

$$NH_{3d} + H_2O \leftrightarrows NH_4OH \leftrightarrows NH_4^+ + OH^-$$

Il est donc possible de contrôler l'équilibre de l'ammoniac dissout (NH3d) par actions simultanées sur :

- La température du méthaniseur
- Le pH du méthaniseur
- La pression partielle en ammoniac gaz dans le ciel du méthaniseur

Or, sur le procédé en voie épaisse, le pH évolue de 6,5 (premier compartiment) à 8,5 (dernier secteur piston). La compartimentation du flux piston à l'intérieur du méthaniseur entraîne aussi une sectorisation du ciel gazeux : ainsi, les ciels gazeux des derniers secteurs sont « riches » en concentration d'ammoniac (température de 55°C et pH compris entre 8 et 8,5).

La température dans ces derniers compartiments est préférentiellement entre 45°C et 60 °C et, plus préférentiellement, entre 50°C et 60°C. Le potentiel hydrogène dans ces derniers compartiments est préférentiellement entre 7,9 à 8,7.

En effet, dans ces conditions, l'équilibre est orienté vers la forme $NH_3d$. C'est une énorme différence par rapport aux autres procédés de méthanisation, ou le ciel gazeux est uniforme en composition car non compartimenté.

Ainsi, sur ces derniers secteurs du réacteur piston, on peut alors faire passer l'ammoniac de la fraction liquide vers la fraction gaz, selon l'équilibre :

$$NH_{3d} \leftrightarrows NH_3g$$

En effet, selon la loi de Henry, la concentration maximale d'un gaz en solution, en équilibre avec une atmosphère contenant ce gaz, est proportionnelle à la pression partielle de ce gaz en ce point :

$$C^s_{NH3d} = p_{NH3g} \times H_{NH3}$$

Avec :

- $C^s_{NH3d}$ : concentration maximale (à saturation) de l'ammoniac dans le liquide
- $p_{NH3g}$ : pression partielle de l'ammoniac dans le gaz
- $H_{NH3}$ : constante de Henry pour l'ammoniac.

Selon cette relation, il est donc nécessaire de diminuer la pression partielle de l'ammoniac dans le ciel gazeux du méthaniseur. Sur le procédé en voie épaisse, cette opération peut facilement être effectuée par la recirculation du biogaz utilisé lors des séquences d'agitation.

La présente invention permet d'éliminer l'ammoniac $NH_3g$ dans la boucle de recirculation, comme décrit en regard des figures 1 et 4.

Une partie du biogaz produit est traitée dans une unité spécifique d'élimination du $NH_3g$, puis est réinjecté sans

NH$_3$ dans le ou les secteurs identifiés. Cette opération a pour effet de stripper le NH$_{3d}$ (NH$_3$d → NH$_3$g) par abaissement de la pression partielle dans le ciel du méthaniseur. Le biogaz enrichi en NH$_3$g est de nouveau traité dans l'unité spécifique d'élimination du NH$_3$g, et le cycle est reproduit jusqu'à obtenir les concentrations désirées en ammoniac dans le liquide et dans le gaz.

Le principe de fonctionnement du procédé d'élimination de l'ammoniac NH$_3$g issu du biogaz est le suivant. L'unité d'absorption gaz/liquide, présenté dans la figure 4, est constituée d'une tour de lavage à l'acide avec présence de garnissage. Le biogaz chargé en NH$_3$g est envoyé à contre-courant d'un flux d'acide afin de favoriser une réaction acido-basique et sa conversion en sel d'ammonium. Par exemple, l'utilisation de l'acide sulfurique (H$_2$SO$_4$) comme éluant a un intérêt agronomique pour les exploitants d'unités puisque le produit de sa réaction conduit à une solution de sulfate d'ammonium (engrais). La récupération de cette solution chargée est effectuée en fond de colonne. Le biogaz ainsi épuré de la fraction ammoniacale est récupéré en tête de colonne de lavage avant d'être réutilisé dans le procédé de méthanisation.

De nombreux travaux universitaires ont déjà décrit ce processus d'élimination de l'ammoniac, travaux tels que :

- Walker, M., Iyer, K., Heaven, S., and Banks, C. J. (2011). "Ammonia removal in anaerobic digestion by biogas stripping: An évaluation of process alternatives using a first order rate model based on experimental findings". Chemical Engineering Journal. 178(15), 138-145; et
- F.Abouelenien, W,Fujiwara, Y.Namba, M.Kosseva N Nishio, Improved methan fermentation of chicken manure via ammonia removal by biogas recycle, Bioresour. Technol. 101 (2010) 6368-6373.

Néanmoins, les procédés actuels ne sont pas adaptés pour réaliser ce traitement in-situ de l'ammoniac. En effet, en raison d'une part d'une absence de compartimentation des ciels gazeux, et d'autre part d'une composition homogène du gaz (induite par un fonctionnement en réacteur parfaitement agité), les volumes de biogaz à traiter sont beaucoup trop importants.

Le procédé en voie épaisse illustré en figure 3, dans ce domaine, de nombreux avantages :

a. Le fonctionnement piston induit une sectorisation dans laquelle les concentrations en NH$_{3d}$ sont différentes (le pH ne dépasse 7,8 que sur la seconde partie du réacteur)

b. Les ciels gazeux sont sectorisés, le pilotage étant effectué par la gestion des titres en méthane et CO$_2$ dans les différents secteurs. L'inventeur a déjà observé que les concentrations en NH$_3$ dans le gaz, sur le pilote industriel et dans les derniers secteurs du réacteur, atteignent des valeurs comprises entre 0,5 et 1% (pour 100 ppm dans les procédés classiques). Le procédé en voie épaisse concentre donc naturellement de l'ammoniac sur des volumes de biogaz assez faibles.

Sur le compartiment d'hydrolyse (1$^{er}$ secteur du réacteur piston), le titre en méthane est préférentiellement compris entre 30% et 45%. Ce titre augmente progressivement sur les secteurs suivants du réacteur piston. Sur le dernier secteur, le titre en méthane est compris dans l'intervalle suivant : entre 52% et 65%. Les titres en CO$_2$ sont compris dans des intervalles complémentaires : entre 70% et 55% pour le compartiment d'hydrolyse, et entre 35% à 48% sur le dernier secteur du réacteur piston.

c. La pression partielle en ammoniac dans le ciel gazeux peut facilement être contrôlée. En effet, on réinjecte nativement du gaz dans le méthaniseur, pour assurer les fonctions de brassage et de sectorisation. Il n'est donc pas nécessaire d'ajouter une source d'injection de gaz, comme cela peut être le cas sur les autres procédés agités mécaniquement.

Le procédé en voie épaisse est donc le procédé préférentiel au sein duquel cette élimination peut être réalisée. La robustesse du fonctionnement de ce procédé en voie épaisse peut ainsi être fortement améliorée, lui donnant un avantage par rapport aux autres technologies voie épaisse. Aujourd'hui reconnu comme un procédé industriel mature, le procédé en voie épaisse devient un procédé présentant une réelle rupture technologique en méthanisation. La présente invention permet de retrouver la robustesse d'un procédé mésophile, tout en restant sur un niveau de température thermophile.

Dans le cas d'une unité de méthanisation en voie liquide infiniment mélangée, l'invention fonctionne d'une manière similaire à ce qui est décrit dans la présente description : le biogaz est injecté dans le liquide du digesteur (par exemple le procédé de brassage au gaz de la société Degremont (marque déposée), sur les digesteurs de traitement des boues issues du traitement des eaux urbaines) et peuvent être traité dans une colonne de lavage gaz, comme décrit ci-dessus, avant d'être réinjecté dans le liquide de digestion.

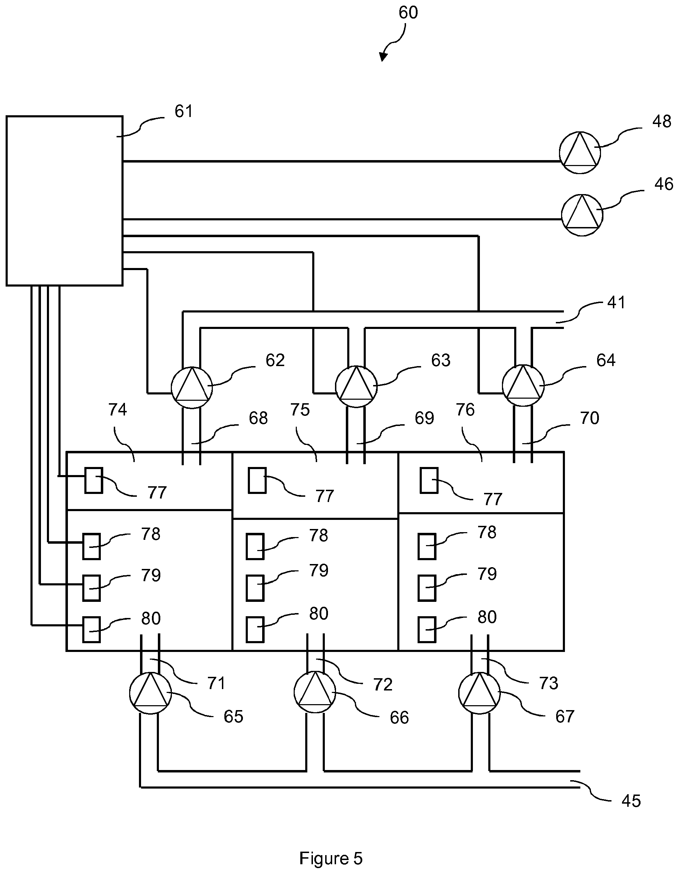

On observe, sur la figure 5, une vue schématique d'un mode de réalisation particulier du dispositif 60 objet de l'invention.

Le dispositif de méthanisation 60 comporte :

- un réacteur de méthanisation comportant, par exemple, trois compartiments 74, 75 et 76 dans lesquels se trouve un substrat en fermentation, produisant une atmosphère comportant au moins du méthane et de l'ammoniac,
- une unité d'extraction de l'atmosphère de chaque compartiment, l'unité comportant :

  - des premiers conduits d'extraction 41 et 68, 69, 70, respectivement, de l'atmosphère des compartiments 74, 75 et 76,
  - une colonne de lavage (voir figure 4) dans laquelle débouche le premier conduit 41 et qui comporte au moins un injecteur d'un liquide d'épuration,
  - des deuxièmes conduits d'extraction 45 du gaz lavé dont la concentration en ammoniac a diminué et d'injection 71, 72 et 73 dudit gaz dans les compartiments 74, 75 et 76, respectivement.

Le substrat, ou matière en fermentation circule successivement du compartiment 74 au compartiment 75 puis au compartiment 76, par exemple sous l'action de pistons (non représentés). Par exemple, dans l'ordre, au sein du compartiment 74 se réalisent d'hydrolyse et acidogenèse, dans le compartiment 75, l'acétogenèse et, dans le compartiment 76, la méthanogenèse.

Le compartiment 74, en amont, est associé à une pompe ou une vanne 65 qui reçoit du gaz de la sortie 45 de la colonne de lavage 42 et qui injecte du gaz dans le deuxième conduit d'injection 71 qui débouche dans le substrat en fermentation. Le compartiment 74 est associé au premier conduit d'extraction 68 muni d'une pompe ou d'une vanne 62 qui délivre du gaz issu de l'atmosphère du compartiment 74 à l'entrée 41 de la colonne de lavage 42.

Le compartiment intermédiaire 75 est associé à une pompe ou une vanne 66 qui reçoit du gaz de la sortie 45 de la colonne de lavage 42 et qui injecte du gaz dans le deuxième conduit d'injection 72 qui débouche dans le substrat en fermentation. Le compartiment 75 est associé au premier conduit d'extraction 69 muni d'une pompe ou d'une vanne 63 qui délivre du gaz issu de l'atmosphère du compartiment 75 à l'entrée 41 de la colonne de lavage 42.

Le compartiment aval 76 est associé à une pompe ou une vanne 67 qui reçoit du gaz de la sortie 45 de la colonne de lavage 42 et qui injecte du gaz dans le deuxième conduit d'injection 73 qui débouche dans le substrat en fermentation. Le compartiment 76 est associé au premier conduit d'extraction 70 muni d'une pompe ou d'une vanne 64 qui délivre du gaz issu de l'atmosphère du compartiment 76 à l'entrée 41 de la colonne de lavage 42.

Chacun des compartiments 74, 75 et 76 comporte des capteurs, physiques ou logiciels, de concentration d'ammoniac dans le substrat 78, de température 79, de pH 80 et de pression de l'atmosphère 77. Un capteur logiciel, ou « observateur d'état » est un programme qui estime une grandeur physique sans la mesurer directement. Il utilise pour cela les informations d'autres capteurs physiques. Un observateur d'état est une extension d'un modèle représenté sous forme de représentation d'état. Lorsque l'état d'un système n'est pas mesurable, on conçoit un observateur qui permet de reconstruire l'état à partir d'un modèle du système dynamique et des mesures d'autres grandeurs.

Les atmosphères des compartiments 74, 75 et 76 sont indépendantes, par exemple parce qu'elles sont dans différentes cuves ou séparées par des cloisons, comme illustré en figure 3.

Les deuxièmes conduits d'injection sont ici représentés comme traversant le plancher des compartiments. En variantes, ils traversent les parois latérales ou le plafond du compartiment, comme illustré en figures 1 et 3.

Une unité de contrôle 61, prenant par exemple la forme d'un serveur reçoit les signaux issus des capteurs 77 à 80 et commande le fonctionnement du dispositif, notamment le fonctionnement des pompes ou vannes 46, 48 et 62 à 67. Les pompes et vannes associées aux différents compartiments sont pilotées indépendamment par l'unité de contrôle 61. Préférentiellement, le gaz lavé injecté dans un compartiment provient du même compartiment.

Préférentiellement, les deuxièmes conduits d'injection 71, 72 et 73 présente au moins un orifice d'injection du gaz proche du fond des compartiments 74, 75 et 76, respectivement.

L'unité de contrôle 61 est configurée pour déclencher le fonctionnement de l'unité d'extraction pour réduire la concentration en ion ammonium lorsque la concentration captée est supérieure 4 g/L de matière en fermentation et, préférentiellement, lorsque la concentration captée est supérieure 3 g/L de matière en fermentation.

La matière à fermenter est infiniment diluée ou présente taux de matière sèche supérieur à 15 %, préférentiellement supérieur à 17 % et, encore plus préférentiellement supérieur à 20 %.

Préférentiellement, au moins un compartiment (notamment le compartiment de méthanogenèse), dans lequel l'unité d'extraction extrait une partie de l'atmosphère et injecte du gaz lavé, présente :

- une température comprise entre 45 (préférentiellement 50°C) et 60 °C,
- un potentiel hydrogène compris entre 7,5 et 8,7 et, plus préférentiellement, compris entre 7,9 et 8,7 et/ou
- une atmosphère présentant un titre en méthane compris entre 52% et 65%.

**Revendications**

1. Dispositif (10) de méthanisation, **caractérisé en ce qu'**il comporte :

   - un réacteur de méthanisation comportant au moins un compartiment (11) dans lequel est inséré un substrat (12) en fermentation, produisant une atmosphère (13) comportant au moins du méthane et de l'ammoniac,
   - une unité d'extraction de l'atmosphère d'au moins un compartiment, l'unité comportant :

      - un premier conduit d'extraction (14) de l'atmosphère d'au moins un compartiment,
      - une colonne de lavage (15) dans laquelle débouche le premier conduit et qui comporte au moins un injecteur (16) d'un liquide d'épuration,
      - au moins un deuxième conduit (17) d'extraction du gaz lavé dont la concentration en ammoniac a diminué et d'injection dudit gaz dans au moins un compartiment.

2. Dispositif (10) de méthanisation selon la revendication 1, dans lequel le liquide d'épuration comporte de l'acide sulfurique, nitrique ou phosphorique.

3. Dispositif de méthanisation selon l'une des revendications 1 ou 2, qui comporte, pour au moins un compartiment, un capteur de taux d'ammoniac dans la matière en fermentation et une unité de contrôle configurée pour déclencher le fonctionnement de l'unité d'extraction pour réduire la concentration en ion ammonium lorsque la concentration captée est supérieure 4 g/L de matière en fermentation.

4. Dispositif de méthanisation selon la revendication 3, dans lequel l'unité de contrôle est configurée pour déclencher le fonctionnement de l'unité d'extraction pour réduire la concentration en ion ammonium lorsque la concentration captée est supérieure 3 g/L de matière en fermentation.

5. Dispositif de méthanisation selon l'une des revendications 1 à 4, qui comporte une pluralité de compartiments de fermentation, dont les atmosphères sont rendues indépendantes en étant séparées par au moins une cloison, chaque compartiment comportant un conduit d'injection (17) de gaz pour lequel la quantité de gaz injecté dans chaque compartiment est régi par une vanne, chaque vanne étant pilotée indépendamment.

6. Dispositif de méthanisation selon l'une des revendications 1 à 5, qui comporte une matière à fermenter dont le taux de matière sèche est supérieur à 15 %, par exemple supérieur à 20 %.

7. Dispositif de méthanisation selon l'une des revendications 1 à 6, qui comporte au moins une cheminée descendant dans la matière en fermentation pour injecter le gaz lavé dans un compartiment d'où le gaz à laver a été extrait par l'unité d'extraction.

8. Dispositif de méthanisation selon la revendication 7, qui comporte un moyen de mise sous pression du gaz à injecter par une cheminée à une pression comprise entre 5 et 8 bars.

9. Dispositif de méthanisation selon l'une des revendications 1 à 8, dans lequel l'unité d'extraction est configurée pour extraire une partie de l'atmosphère dans un compartiment où se trouvent la majorité des bactéries acidogènes.

10. Dispositif de méthanisation selon l'une des revendications 1 à 9, dans lequel l'unité d'extraction est configurée pour extraire une partie de l'atmosphère dans un compartiment où est effectuée la méthanogenèse.

11. Dispositif de méthanisation selon l'une des revendications 1 à 10, dans lequel au moins un compartiment dans lequel l'unité d'extraction extrait une partie de l'atmosphère présente une température comprise entre 45°C et 60 °C.

12. Dispositif de méthanisation selon l'une des revendications 1 à 11, dans lequel au moins un compartiment dans lequel l'unité d'extraction extrait une partie de l'atmosphère présente un potentiel hydrogène compris entre 7,5 et 8,7, par exemple entre 7,9 et 8,7.

13. Dispositif de méthanisation selon l'une des revendications 1 à 12, dans lequel au moins un compartiment dans lequel l'unité d'extraction extrait une partie de l'atmosphère présente un titre en méthane compris entre 52% et 65%.

14. Procédé (20) de méthanisation, **caractérisé en ce qu'**il comporte les étapes suivantes :

- réaction (21) de méthanisation par fermentation d'un substrat (12) dans au moins un compartiment (11), la réaction produisant une atmosphère (13) comportant au moins du méthane et de l'ammoniac,
- extraction (22) de l'atmosphère d'au moins un compartiment,
- lavage (23) de l'atmosphère extraite par injection d'un liquide d'épuration,
- extraction (24) du gaz lavé dont la concentration en ammoniac a diminué et
- injection (25) dudit gaz dans au moins un compartiment.

**Patentansprüche**

1. Methanisierungsvorrichtung (10), **dadurch gekennzeichnet, dass** sie Folgendes umfasst:

   - einen Methanisierungsreaktor mit mindestens einer Kammer (11), in die ein fermentierendes Substrat (12) eingeführt wird, wodurch eine Atmosphäre (13) erzeugt wird, die mindestens Methan und Ammoniak enthält,
   - eine Vorrichtung zum Abzug der Atmosphäre aus mindestens einer Kammer, wobei diese Vorrichtung Folgendes umfasst:

     - ein erste Abzugsleitung (14) für den Abzug der Atmosphäre von mindenstens einer Kammer,
     - eine Waschsäule (15), in die die erste Leitung mündet und die mindestens einen Injektor (16) einer Reinigungsflüssigkeit beinhaltet,
     - mindestens eine zweite Leitung (17) zum Abzug des gewaschenen Gases mit verminderter Ammoniakkonzentration und zur Einspritzung des genannten Gases in mindestens eine Kammer.

2. Methanisierungsvorrichtung (10) nach Anspruch 1, bei der die Reinigungsflüssigkeit Schwefelsäure, Salpetersäure oder Phosphorsäure enthält.

3. Methanisierungsvorrichtung nach einem der Ansprüche 1 oder 2, die für mindestens eine Kammer einen Ammoniakgehalt-Sensor im Fermentationsmaterial und eine Kontrollvorrichtung aufweist, die so konfiguriert ist, dass sie die Abzugseinrichtung in Betrieb setzt, um die Konzentration an Ammoniumionen zu reduzieren, wenn die gemessene Konzentration höher als 4 g/l Fermentationsmaterial ist.

4. Methanisierungsvorrichtung nach Anspruch 3, bei der die Kontrollvorrichtung so konfiguriert ist, dass sie die Abzugseinrichtung in Betrieb setzt, um die Konzentration an Ammoniumionen zu reduzieren, wenn die gemessene Konzentration höher als 3 g/l Fermentationsmaterial ist.

5. Methanisierungsvorrichtung nach einem der Ansprüche 1 bis 4, die eine Vielzahl von Fermentationskammern umfasst, deren Atmosphären voneinander unabhängig gemacht werden, indem sie durch mindestens eine Trennwand voneinander getrennt werden, wobei jede Kammer eine Leitung für die Einspritzung (17) von Gas aufweist und die in jede Kammer eingespritzte Gasmenge durch ein Ventil geregelt wird, wobei jedes Ventil unabhängig gesteuert wird.

6. Methanisierungsvorrichtung nach einem der Ansprüche 1 bis 5, die ein Fermentationsmaterial mit einem Trockensubstanzgehalt von mehr als 15 %, beispielsweise mehr als 20 %, aufweist.

7. Methanisierungsvorrichtung nach einem der Ansprüche 1 bis 6, die mindestens ein in das Fermentationsmaterial eintauchendes Rohr zum Einspritzen des gewaschenen Gases in eine Kammer aufweist, aus der das zu waschende Gas zuvor von der Abzugseinrichtung entzogen wurde.

8. Methanisierungsvorrichtung nach Anspruch 7, die eine Einrichtung zur Druckbeaufschlagung des durch ein Rohr einzuspritzenden Gases auf einen Druck zwischen 5 und 8 bar aufweist.

9. Methanisierungsvorrichtung nach einem der Ansprüche 1 bis 8, bei der die Abzugseinrichtung so konfiguriert ist, dass sie einen Teil der Atmosphäre aus einer Kammer, in der sich die meisten acidogenen Bakterien befinden, entzieht.

10. Methanisierungsvorrichtung nach einem der Ansprüche 1 bis 9, bei der die Abzugseinrichtung so konfiguriert ist, dass sie einen Teil der Atmosphäre aus einer Kammer, in der die Methogenese stattfindet, entzieht.

**11.** Methanisierungsvorrichtung nach einem der Ansprüche 1 bis 10, bei der mindestens eine Kammer, aus der die Abzugseinrichtung einen Teil der Atmosphäre entzieht, eine Temperatur zwischen 45 °C und 60 °C aufweist.

**12.** Methanisierungsvorrichtung nach einem der Ansprüche 1 bis 11, bei der mindestens eine Kammer, aus der die Abzugseinrichtung einen Teil der Atmosphäre entzieht, ein Wasserstoffpotential zwischen 7,5 und 8,7, beispielsweise zwischen 7,9 und 8,7 aufweist.

**13.** Methanisierungsvorrichtung nach einem der Ansprüche 1 bis 12, bei der mindestens eine Kammer, aus der dieAbzugseinrichtung einen Teil der Atmosphäre entzieht, einen Methangehalt zwischen 52 % und 65 % aufweist.

**14.** Verfahren (20) zur Methanisierung, **dadurch gekennzeichnet, dass** es die folgenden Schritte umfasst:

- Methanisierungsreaktion (21) durch Fermentation eines Substrats (12) in mindestens einer Kammer (11), wobei die Reaktion eine Atmosphäre (13) erzeugt, die mindestens Methan und Ammoniak enthält,
- Entziehen (22) der Atmosphäre mindestens einer Kammer,
- Waschen (23) der entzogenen Atmosphäre durch Einspritzung einer Reinigungsflüssigkeit,
- Entziehen (24) des gewaschenen Gases, dessen Ammoniakkonzentration sich verringert hat und
- Einspritzen (25) des Gases in mindestens eine Kammer.

**Claims**

**1.** Methanization device (10), **characterized in that** it comprises:

- a methanization reactor comprising at least one compartment (11) into which a fermentation substrate (12) is inserted, producing an atmosphere (13) comprising at least methane and ammonia;
- a unit for extracting the atmosphere from at least one compartment, said unit comprising:

  - a first duct (14) for extracting atmosphere from at least one compartment;
  - a washing column (15) into which the first duct emerges and which comprises at least one injector (16) of a purification liquid;
  - at least one second duct (17) for extracting the washed gas whose ammonia concentration has decreased and for injecting said gas into at least one compartment.

**2.** Methanization device (10) according to claim 1, wherein the purification liquid comprises sulfuric, nitric or phosphoric acid.

**3.** Methanization device according to one of claims 1 or 2, which comprises, for at least one compartment, a sensor of the ammonia level in the fermentation material, and a control unit configured to trigger the operation of the extraction unit to reduce the ammonium ion concentration when the concentration measured by the sensor is above 4 g/L of fermentation material.

**4.** Methanization device according to claim 3, wherein the control unit is configured to trigger the operation of the extraction unit to reduce the ammonium ion concentration when the concentration measured by the sensor is above 3 g/L of fermentation material.

**5.** Methanization device according to one of claims 1 to 4, which comprises a plurality of fermentation compartments, whose atmospheres are made independent through being separated by at least one partition, each compartment comprising a gas injection duct (17) for which the quantity of gas injected into each compartment is governed by a valve, each valve being controlled independently.

**6.** Methanization device according to one of claims 1 to 5, which comprises a fermentation material whose dry matter content is higher than 15%, for example higher than 20%.

**7.** Methanization device according to one of claims 1 to 6, which comprises at least one flue descending into the fermentation material for injecting the washed gas into a compartment from which the gas to be washed has been extracted by the extraction unit.

8. Methanization device according to claim 7, which comprises a means for pressurizing the gas to be injected by a flue to a pressure between 5 and 8 bar.

9. Methanization device according to one of claims 1 to 8, wherein the extraction unit is configured to extract a portion of the atmosphere from a compartment in which most of the acid-producing bacteria are located.

10. Methanization device according to one of claims 1 to 9, wherein the extraction unit is configured to extract a portion of the atmosphere from a compartment in which methanogenesis is performed.

11. Methanization device according to one of claims 1 to 10, wherein the temperature in at least one compartment from which the extraction unit extracts a portion of the atmosphere is between 45°C and 60°C.

12. Methanization device according to one of claims 1 to 11, wherein the potential hydrogen of at least one compartment from which the extraction unit extracts a portion of the atmosphere is between 7.5 and 8.7, for example between 7.9 and 8.7.

13. Methanization device according to one of claims 1 to 12, wherein the methane content of at least one compartment from which the extraction unit extracts a portion of the atmosphere is between 52% and 65%.

14. Methanization method (20), **characterized in that** it comprises the following steps:

   - methanization reaction (21) by fermentation of a substrate (12) in at least one compartment (11), the reaction producing an atmosphere (13) comprising at least methane and ammonia;
   - extracting (22) the atmosphere from at least one compartment;
   - washing (23) the extracted atmosphere by injecting a purification liquid;
   - extracting (24) the washed gas whose ammonia concentration has decreased; and
   - injecting (25) said gas into at least one compartment.

Figure 1

| | |
|---|---|
| Réaction de méthanisation par fermentation d'un substrat | 21 |
| Extraction de l'atmosphère de la cuve | 22 |
| Lavage de l'atmosphère de la cuve | 23 |
| Extraction du gaz lavé | 24 |
| Injection du gaz lavé dans la cuve | 25 |

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1811015 A
- FR 2013051938 W
- WO 2014027165 A

**Littérature non-brevet citée dans la description**

- **BUSWELL, A. M. ; H. F. MUELLER.** Mechanism of methane fermentation. *Industrial and Engineering Chemistry,* 1952, vol. 44 (3), 550-552
- **WALKER, M. ; LYER, K. ; HEAVEN, S. ; BANKS, C. J.** Ammonia removal in anaerobic digestion by biogas stripping: An évaluation of process alternatives using a first order rate model based on experimental findings. *Chemical Engineering Journal,* 2011, vol. 178 (15), 138-145
- **F.ABOUELENIEN ; W,FUJIWARA ; Y.NAMBA ; M.KOSSEVA ; N NISHIO.** Improved methan fermentation of chicken manure via ammonia removal by biogas recycle. *Bioresour. Technol.,* 2010, vol. 101, 6368-6373